# EUROPEAN PATENT APPLICATION

(11) **EP 2 866 153 A1**
(43) Date of publication of application: **29.04.2015**
(21) Application number: 13189734.0
(22) Date of filing: 22.10.2013
(51) Int. Cl.: G06F 17/24, G10L 15/26

(54) **Speech recognition method and system with simultaneous text editing**

(71) Applicant: Agfa Healthcare, 2640 Mortsel (BE)
(72) Inventor: Vanheuverswyn, Jeroen, 2640 Mortsel (BE); Renard, Guy, 2640 Mortsel (BE)
(74) Representative: Verbrugghe, Anne Marie L.

(57) **Abstract**

In order to generate text from audio input, speech from a user is stored in an audio queue (201), stored speech is transformed into text through speech recognition, and the text is displayed to the user. A text editing event (106, 107) inputted by the user is also stored in the audio queue (201), and changes resulting from the text editing event (201) are instantly displaying to the user. When all speech queued prior to the text editing event (107) in the audio queue (201) is transformed into text, speech recognition is halted and the text editing event is processed (111) while additional speech from the user is stored in the audio queue (201). As soon as the text editing event has been processed, speech recognition is resumed.

## Description

### Field of the Invention

The present invention generally relates to a method and system for transforming speech, i.e. dictated words, into written text. Tools used in such method or system are generally known as dictation tools. The invention in particular concerns a more user-friendly method and system that allows editing of the text while converting speech into text.

### Background of the Invention

Dictation tools that convert speech or dictated words into written text are used in a wide variety of applications. One example is the creation of medical reports. The authors of such reports, e.g. radiologists, cardiologists, technologists, etc., use speech recognition to fill out certain fields in a medical report with predefined format and text. The user dictates the words, these words are recognized by a voice recognition engine and transformed into text that is inserted in the selected field.

Existing dictation tools typically have a recording mode wherein speech is recorded and transformed into text, and an editing mode wherein the written text can be edited. If a user desires to manipulate text, e.g. select a portion of text, delete words, over-dictate a group of words in a sentence, etc., the recording mode must be stopped, the editing mode must be started, the text manipulations must be executed in the editing mode, and the recording mode must be re-started once the text editing is done. The recording button that allows to restart the recording mode must be clicked a lot, in particular when multiple text manipulations are needed, as a result of which existing dictation tools are perceived as non-user-friendly.

European patent application EP 2 261 893 recognizes in paragraph [0003] that the modal behaviour of existing dictation systems is ineffective since correction of a word requires too many actions or clicks from the user. EP 2 261 893 consequently describes a system for converting audio into text with a recording mode, called dictation mode, wherein speech is queued, a synchronous reproduction mode wherein text is displayed while the speech is played back enabling the user to review the text, and an editing mode wherein the user can correct words in the text. In EP 2 261 893, the modal behaviour of the system is improved by enabling editing the text during the synchronous reproduction mode. The user however still has to interrupt the dictation mode each time a text manipulation is desired. This slows down report creation.

It is an objective of the present invention to disclose a method and system for generating written text from inputted speech that resolves the shortcomings of prior art solutions identified here above. More particularly, it is an objective to define a method and system that increases user-friendliness and substantially speeds up report creation through voice recognition.

### Summary of the Invention

According to the present invention, the above defined objective is realized by the method for generating and editing text from audio input defined by claim 1, the method comprising:
- queuing speech from a user in an audio queue;
- transforming the speech stored in the audio queue into text through speech recognition;
- displaying the text to the user;
- queuing a text editing event in the audio queue;
- instantly displaying to the user changes resulting from the text editing event;
- halting the speech recognition when all speech queued prior to the text editing event in the audio queue is transformed;
- processing the text editing event and editing the text while queuing additional speech from the user in the audio queue; and
- resuming the speech recognition when the text editing event has been processed.

Thus, the invention enables the user to edit the text while he/she is in speech recording mode. While recording additional speech in the audio queue, the user can re-position the cursor in the text displayed, select portions of the displayed text, delete portions of the displayed text, over-dictate selected text portions, etc. Speech will continuously be recorded in the audio queue while text manipulations resulting from editing events are made visible instantly in the displayed text. In case of repositioning of the cursor for instance, the cursor is already visually moved to the new position in the displayed text while dictated speech that is still being converted into text, is added to the previous position. As soon as all speech dictated and recorded prior to the text editing event is converted into written text that is displayed, the queued text editing event is processed. As a result thereof, the speech recognition engine will be informed on the changes in the text resulting from the text editing event. Additional speech that is dictated while the text editing event is processed, is in the meantime recorded in the audio queue. Speech recognition is halted as long as the text editing event is being processed and resumed again automatically as soon as the text editing event has been processed.

The method according to the invention significantly enhances the user-friendliness of dictation tools since the user no longer has to switch between recording mode and editing mode. Excessive button clicks or other manual mode switch instructions are thus avoided. The user starts recording once and stops recording once. In between, button clicks, keystrokes, mouse clicks or screen touches are only required for text manipulations, not to switch modes. Since the user can edit or correct his report while dictating additional words, the present invention also significantly speeds up report creation.

According to an optional aspect of the method according to the present invention, the text editing event comprises a voice command.

Indeed, the text editing events may be entered through button clicks, keystrokes, mouse clicks, screen touches or through the use of other peripheral devices. Alternatively however, a text editing event may be inputted through voice commands in between the dictated words that are converted to text. When such voice command is recognized by the speech recognition engine, the voice command is queued into the audio queue whereas the changes resulting from the voice command are instantly displayed. As soon as all speech recorded in the audio queue prior to the voice command is transformed into displayed text, the voice command is processed and the speech recognition engine is informed of the changes resulting from the voice command. During the processing of the voice command, speech recognition is halted.

In accordance with a further optional aspect of the method according to the present invention, the text editing event comprises one or more of:
- a navigation instruction in the text;
- a select and edit instruction for a part of the text;
- a select and format instruction for part of the text;
- a select and delete instruction for part of the text;
- a select instruction for a field value from a drop-down list;
- an instruction to insert a predefined text portion into the text; and
- a deselect instruction for part of the text that has been selected.

In addition to a method as defined by claim 1, the present invention also relates to a corresponding system for generating and editing text from audio input, the system comprising:
- an audio queue configured to store speech from a user;
- a speech recognition engine configured to transform the speech stored in the audio queue into text;
- a user view engine and display for displaying the text to the user; and
- an event processor for processing a text editing event inputted by the user, wherein
- the audio queue is adapted to queue the text editing event;
- the user view engine and display are adapted to instantly display to the user changes resulting from the text editing event;
- the event processor is adapted to halt speech recognition by the speech recognition engine when all speech queued prior to the text editing event in the audio queue is transformed;
- the event processor is further configured to process the text editing event and edit the text while additional speech from the user is stored in the audio queue; and
- the event processor is adapted to resume speech recognition by the speech recognition engine when the text editing event has been processed.

### Brief Description of the Drawings

Fig. 1 illustrates the communication flow between speech recognition engine and user view engine in an embodiment of the present invention;

Fig. 2 is a functional block scheme of an embodiment of the system for generating and editing text from audio input according to the present invention; and

Fig. 3A-3G illustrate evolution of the user view and speech configuration engine view in an embodiment of the present invention.

### Detailed Description of Embodiment(s)

The invention enables the user of a dictation tool to simultaneously record speech and edit displayed text by queuing each user editing action in the text into the audio queue. The changes resulting from an editing action in the text are made instantly visible to the user but the actual processing of the user editing action and altering of the speech recognition engine's view on the text is done later by queuing the user editing action in the audio queue. Thus, the view of the user, i.e. the text as displayed to the user, and the speech recognition engine view, i.e. the text as known by the speech recognition engine, can differ at a certain point in time.

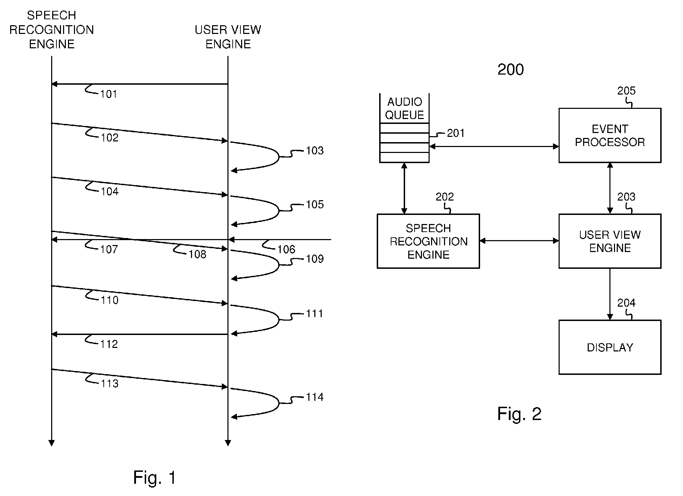

Fig. 1 shows the communication flow between the speech recognition engine 202 and the user view engine 203 in the embodiment 200 of the system according to the present invention shown in Fig. 2 at a point in time when the user performs a single text editing event while speech recording is ongoing. The subsequent steps are explained in detail in the following paragraphs with interleaved reference to Fig. 1 and Fig. 2.

In a first step, it is assumed that the user has activated the recording of speech. This is done for instance by clicking a button in the graphical user interface displayed on display 204. The user view engine 203 informs the speech recognition engine 202 that recording is started as is indicated by arrow 101 in Fig. 1.

The user, for instance the report author in case a report is filled out, starts the speech recording mode with one button click. He/she can then dictate words which are immediately converted into written text in a report that is shown on display 204.

In the recording mode, recorded audio is stored in audio queue 201 and transformed into text through automated speech recognition performed by the speech recognition engine 202. The speech-to-text transformed words are delivered by the speech recognition engine 202 to the user view engine 203 as indicated by arrows 102 and 104 in Fig. 1, and the text is processed by the user view engine 203 for presentation to the user on display 204 as is indicated by arrows 103 and 105 in Fig. 1.

While in speech recording mode, the user can dictate text, but he/she can also perform text editing actions like:
- dictating voice commands;
- repositioning the cursor in the displayed report;
- selecting or deselecting portions of text in the displayed report;
- manually typing text;
- applying formatting to selected text portions in the displayed report;
- changing the outline of text portions in the displayed report;
- deleting text portions in the displayed report;
- inserting a predefined text portion in the displayed report;
- selecting a value from a drop-down field;
- etc.

Arrow 106 in Fig. 1 indicates that the user performs such an action on the text shown in display 204. The action is detected by the user view engine 203 and reported to the speech recognition engine 202 and/or the audio queue 201, as indicated by arrow 107 in Fig. 1. This triggers the queuing of a text editing event in the audio queue 201. It is noticed here that although the audio queue 201 and the speech recognition engine 202 are drawn as separate components in Fig. 2, they may be integrated in various embodiments of the invention, and at least in the communication flow scheme drawn in Fig. 1, are assumed to be integrated.

Additional words that were recorded in the audio queue 201 before text editing event 107, are transformed into text and provided by the speech recognition engine 202 to the user view engine 203 as is indicated by arrow 108 in Fig. 1. The text is processed by the user view engine 203 for display, as is indicated by arrow 109 in Fig. 1, and presented to the user. Changes that result from the user action 106 however are instantly displayed by the user view engine 203 and thus made visible to the user immediately.

In summary, if not all dictated words are converted to text yet when the user repositions the cursor and/or edits selected text portions, the cursor in display 204 is already visually moved to the newly selected position while converted text is still being added to the previous cursor position. When all words queued prior to the text editing event 107 in audio queue 201 are converted, the queued text editing event will be processed by event processor 205 and the changes resulting therefrom are reported to the speech recognition engine 202.

The speech recognition engine 202 requires that the text representation of the report whereto converted text is added doesn't change during the addition. Hence no text editing actions are allowed on the version of the text viewed by the speech recognition engine 202. This includes repositioning of the cursor. Consequently, the inputted audio is processed up to the insertion of the text editing event 107. Thereupon, speech recognition by the speech recognition engine 202 is halted and the text editing event 107 is processed. During the processing of the text editing event 107 by the speech recognition engine 202, the user can continue to dictate new words. These words will continuously be recorded in the audio queue 201 such that the user has the impression that he/she can simultaneously dictate speech and edit text that has already been speech-to-text transformed.

As is indicated by arrow 110, the audio queue 201 which is assumed to be integrated with the speech recognition engine instructs the event processor 205 to process the text editing event. It is noticed here that the event processor 205, although drawn as a separate component in Fig. 2, may be integrated with the user view engine 203 in various embodiments of the invention, and at least in the communication flow drawn in Fig. 1 is assumed to be integrated therewith. The text editing event is processed by the event processor 205 as is indicated by arrow 111 in Fig. 1. Thereupon, feedback is provided to the speech recognition engine 202, as indicated by arrow 112 in Fig. 1, and speech recognition is resumed by the speech recognition engine 202.

Audio that was recorded in the audio queue 201 while the text editing event was processed or thereafter, is speech-to-text transformed and the recognized words or written text is reported to the user view engine 203, as is indicated by arrow 113, to be processed for display, as is indicated by arrow 114. The changes applied as a result of the text editing event processing can influence the recognition results when recognition is resumed.

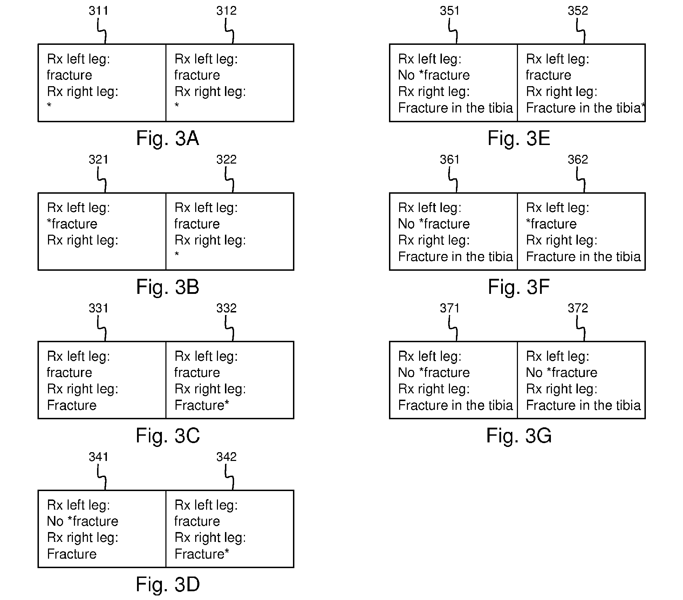

For a particular example wherein a physiologist completes a report on a radio scan of a patient's legs, Fig. 3A-3G illustrate evolution of the text version displayed and seen by the user on the left side, i.e. 311, 321, 331, 341, 351, 361 and 371, and evolution of the text version seen by the speech recognition engine 202 on the right side, i.e. 312, 322, 332, 342, 352, 362 and 372.

In Fig. 3A, the user view 311 and the speech recognition engine's view 312 on the text are identical. It is assumed that the physician has already entered the word "fracture" in the field "Rx left leg" through speech recognition. The asterisk "*" shows the position of the cursor, which is also identical in the user view 311 and the speech recognition engine's view 312. The cursor position is on the fourth line in the report, i.e. in the field "Rx right leg".

It is then assumed that the physician dictates the words "Fracture in the tibia" and clicks on the second line in the report, i.e. below the text "Rx left leg". At this moment there is a first event queued in the audio queue of the speech recognition engine. The repositioning of the cursor "*" however is made visible instantly in the user view 321 as a result of which the user view 321 and the speech recognition engine's view 322 differ in Fig. 3B.

In Fig. 3C, the speech recognition engine recognizes the word "Fracture". This is processed in the user view 331 but the user keeps seeing the cursor "*" at the location where he/she placed it while the word "Fracture" is added to the old position of the cursor, i.e. the position of the cursor in the speech recognition engine's view 322.

In Fig. 3D, the user manually types "No". This is made visible instantly in the user view 341 while the speech recognition engine's view remains unaltered. The manual entry by the user however causes a second event to be queued in the audio queue of the speech recognition engine.

Fig. 3E shows that the audio inputted by the physician up to the first event is processed. The speech recognition engine recognizes the additional words "in the tibia", adds these words to the speech recognition engine's view 352 at the cursor position, and reports the change to the user view engine to be processed in the user view 351.

Thereafter the first event, i.e. repositioning of the cursor by the physician, is encountered in the audio queue. This event is processed by the event processor which will inform the speech recognition engine that the cursor position has changed. The user view 361 will not change, but the position of the cursor "*" in the speech recognition engine's view 362 is updated as a result of the event processing. This is shown in Fig. 3F.

At last, as illustrated by Fig. 3G, the second event in the audio queue is encountered, i.e. the manual entry of the word "No". Again, the event processor shall process this text editing event and inform the speech recognition engine that "No" is inserted. Whereas the user view 371 will remain unchanged, the speech recognition engine's view 372 will be adjusted as a result of which both views become identical again in Fig. 3G.

Fig. 3A-3G illustrate that a physician making use of the system or method according to the present invention can simultaneously dictate words to be inserted in the "Rx right leg" field of the report and correct the text that has been inserted earlier in the "Rx left leg" field. The physician consequently saves time, and superfluous clicks to transit between recording mode and editing mode are avoided, enhancing the overall user-friendliness for the physician.

It is noticed that the method according to the present invention or certain steps thereof shall typically be computer-implemented to run on a data processing system or computing device. A data processing system or computing device that is operated according to the present invention can include a workstation, a server, a laptop, a desktop, a hand-held device, a mobile device, a tablet computer, or other computing device, as would be understood by those of skill in the art.

The data processing system or computing device can include a bus or network for connectivity between several components, directly or indirectly, a memory or database, one or more processors, input/output ports, a power supply, etc. One of skill in the art will appreciate that the bus or network can include one or more busses, such as an address bus, a data bus, or any combination thereof, or can include one or more network links. One of skill in the art additionally will appreciate that, depending on the intended applications and uses of a particular embodiment, multiple of these components can be implemented by a single device. Similarly, in some instances, a single component can be implemented by multiple devices.

The data processing system or computing device can include or interact with a variety of computer-readable media. For example, computer-readable media can include Random Access Memory (RAM), Read Only Memory (ROM), Electronically Erasable Programmable Read Only Memory (EEPROM), flash memory or other memory technologies, CDROM, digital versatile disks (DVD) or other optical or holographic media, magnetic cassettes, magnetic tape, magnetic disk storage or other magnetic storage devices that can be used to encode information and can be accessed by the data processing system or computing device.

The memory can include computer-storage media in the form of volatile and/or nonvolatile memory. The memory may be removable, non-removable, or any combination thereof. Exemplary hardware devices are devices such as hard drives, solid-state memory, optical-disc drives, or the like. The data processing system or computing device can include one or more processors that read data from components such as the memory, the various I/O components, etc.

The I/O ports can allow the data processing system or computing device to be logically coupled to other devices, such as I/O components. Some of the I/O components can be built into the computing device. Examples of such I/O components include a microphone, joystick, recording device, game pad, satellite dish, scanner, printer, wireless device, networking device, or the like.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A method for generating and editing text from audio input, said method comprising:
- queuing speech from a user in an audio queue (201);
- transforming said speech stored in said audio queue (201) into text through speech recognition; and
- displaying said text to said user,
**CHARACTERISED IN THAT** said method further comprises:
- queuing a text editing event (106, 107) in said audio queue (201);
- instantly displaying to said user changes resulting from said text editing event (106);
- halting said speech recognition when all speech queued prior to said text editing event (107) in said audio queue (201) is transformed;
- processing (111) said text editing event and editing said text while queuing additional speech from said user in said audio queue (201); and
- resuming said speech recognition when said text editing event has been processed.

2. A method according to claim 1, wherein said text editing event comprises a voice command.

3. A method according to claim 1 or claim 2, wherein said text editing event comprises one or more of:
- a navigation instruction in said text;
- a select and edit instruction for a part of said text;
- a select and format instruction for part of said text;
- a select and delete instruction for part of said text;
- a select instruction for a field value from a drop-down list;
- an instruction to insert a predefined text portion into said text; and
- a deselect instruction for part of said text that has been selected.

4. A system for generating and editing text from audio input, said system comprising:
- an audio queue (201) configured to store speech from a user;
- a speech recognition engine (202) configured to transform said speech stored in said audio queue (201) into text;
- a user view engine (203) and display (204) for displaying said text to said user; and
- an event processor (205) for processing a text editing event inputted by said user,
**CHARACTERIZED IN THAT**
- said audio queue (201) is adapted to queue said text editing event;
- said user view engine (203) and display (204) are adapted to instantly display to said user changes resulting from said text editing event;
- said event processor (205) is adapted to halt speech recognition by said speech recognition engine (202) when all speech queued prior to said text editing event in said audio queue (201) is transformed;
- said event processor (205) is further configured to process said text editing event and edit said text while additional speech from said user is stored in said audio queue (201); and
- said event processor (205) is adapted to resume speech recognition by said speech recognition engine (202) when said text editing event has been processed.
